# EUROPEAN PATENT SPECIFICATION

(11) **EP 1 356 777 B1**
(45) Date of publication and mention of the grant of the patent: **23.01.2008**
(21) Application number: 03007997.4
(22) Date of filing: 10.04.2003
(51) Int. Cl.: A61B 17/74, A61B 17/17, A61B 17/72

(54) **Screw Guide for an intramedullary nail**
Schraubenführung für einen Marknagel
Guide de vissage pour clou intramedullaire

(30) Priority: 15.04.2002 IT BO20020200
(43) Date of publication of application: 29.10.2003
(73) Proprietor: HIT MEDICA S.r.L., 47900 Rimini (IT)
(72) Inventor: Fontana, Maurizio, 40054 Budrio (Prov. of Bologna) (IT)
(74) Representative: Modiano, Micaela Nadia

(56) References cited:
- EP-A- 0 273 872
- EP-A- 0 696 441
- EP-A- 1 344 494
- WO-A-02/30258
- WO-A-03/041595
- US-A- 5 776 194
- US-A- 5 935 127

## Description

The present invention relates to a device for positioning a screw to be associated with an intramedullary nail inserted in the medullary canal of the femur, particularly for the treatment of fractures that have a fissure located in the subtrochanteric region.

In the particular field of the treatment of femoral fractures, it is known and common practice to insert in the femur, according to EP-A-0 838 199 and Italian Utility Model Patent No. 252187 by the same Applicant, an intramedullary nail that allows to set fractures in various regions of the femur.

US-A-5 935 127 discloses an intramedullary nail having a long axis extending between a proximal end and a distal end, with a middle portion spanning a fracture of a femur bone. At the distal end there are arranged a pair of fastener bores receiving a pair of parallel distal end attachment screws, and at the proximal end there is provided a slot filled with a solid resorbable material, in which the slot extends for a certain distance along the direction of the long axis to provide an increased fastening area for the insertion in the slot with resorbable material of a pair of parallel proximal end attachment screws, by means of a drilling jig having bores fixable in a desired angular location with respect to a line perpendicular to the long axis.

EP-A-0 273 872 discloses an intramedullary nail, having a longitudinal slot for providing flexural elasticity, and distal end holes and proximal end slots receiving fixing screws of pins. An L-shaped guide bracket has inclined guide bushes and a perpendicular guide bush for selectively inserting the proximal screws in the proximal slot.

US-A-5 776 194 discloses a modular intramedullary rod including a stem member and an extension member mutually forming a respective angle. The stem member includes a distal end and a proximal end respectively provided with a distal transverse passage and a proximal transverse passage through which interlocking screws may be removably screwed for securing the stem member to the humerus. The extension member includes one or more transverse passages through which there are removably engaged stabilizing screws. A screw alignment guide is provided for installing the modular intermedullary rod, which is attaches to the extension member and adjusts along three axes for positioning, aligning, and centering the passages in the extension member.

WO 03/041595 A, a document within the meaning of Article 54(3) EPC, discloses a targeting device for a fracture pin having a plurality of pin cross drill holes. The targeting device comprises a targeting arm with one end detachable connected to the proximal end of the fracture pin and the other free end supporting an adjustable targeting head having a plurality of target drill holes alignable with the pin cross drill holes.

EP-A-1 344 494, a document within the meaning of Article 54(3) EPC, discloses a targeting apparatus having a mounting portion for connection to an end of a bone locking nail, a targeting arm, a U-shaped or L-shaped connection portion fixedly connecting the targeting arm to the mounting portion, a cylindrical portion in the lower distal region of the targeting arm, a series of cross-bores provided on the cylindrical portion and alignable with cross-bores of the bone nail, and a rotatable sleeve with a plurality of cross-apertures and rotatably supported about the cylindrical portion at the distal end of the targeting arm in predetermined rotational positions such that at least one aperture is oriented to a bore of the targeting arm for guiding a drill selectively into the cross-bores of the bone nail.

It is also common practice to insert a screw, commonly known as head screw, for treating fractures of the trochanteric region and of the neck of the femur; such screw is inserted so that its axis lies substantially along the anatomical protrusion of the head in order to connect its fragments.

It is often necessary to be able to position additional screws at fissures of a fracture that frequently occur in certain positions that are rather awkward to reach with conventional devices.

For example, fracture fissures located in the subtrochanteric region of the femur are difficult to treat, also because it is likely, in positioning a screw in this region, to incur in unwanted interference with a head screw if a prior one has been inserted earlier.

Moreover, the subtrochanteric fracture fissure can occur with different inclinations with respect to the axis of the femur: accordingly, it is extremely difficult to insert the screw for fixing the fragments of the femur in the optimum direction, which indeed changes in every situation.

The aim of the present invention is to obviate these drawbacks by providing a device that allows to apply easily, and in association with the intramedullary nail, a screw for fixing fragments of the femur formed by a fracture fissure having any inclination.

Within this aim, an object of the present invention is to provide a device that allows to implant a fixing screw in the subtrochanteric region even in the direct vicinity of the head screw.

Another object of the present invention is to achieve this aim with a structure that is simple, relatively easy to provide in practice, safe in use, effective in operation, and relatively low in cost.

In accordance with the invention, there is provided an intramedullary nail with a bone fragment connection screw and a femur head screw, in combination with a device for positioning the bone fragment connection screw connected with the intramedullary nail, as defined in the appended claims. In particular, there is provided an oblique hole and femur head screw at the proximal end of the nail, and a transverse slot and bone fragment connection screw at the same proximal end of the nail below such oblique hole and femur head screw, and in which such transverse slot and bone fragment connection screw have a longitudinal centerline plane which is inclined with respect to the axis of such oblique hole and femur head screw, for advantageously allowing to efficiently and effectively reduce bone fractures located in the femur subtronchanteric region.

Further features and advantages of the invention will become better apparent from the detailed description of a preferred but not exclusive embodiment of a device for positioning a screw to be associated with an intramedullary nail inserted in the medullary canal of the femur according to the invention, illustrated by way of non-limitative example in the accompanying drawings, wherein:

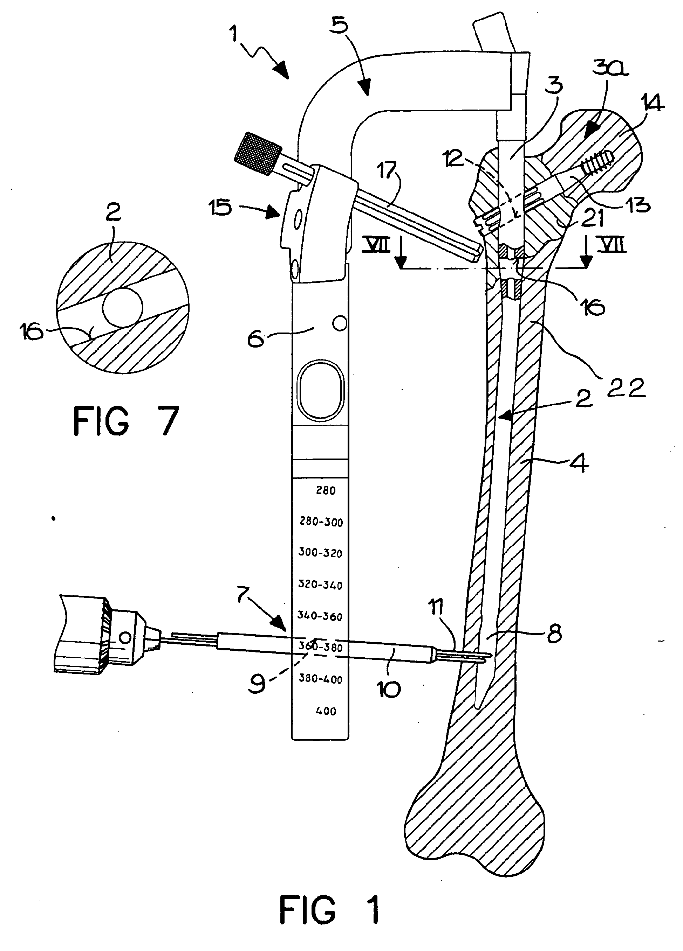

Figure 1 is a partially sectional front view of the device according to the invention, fixed along the outer portion of the arm connected to the upper end of the intramedullary nail;

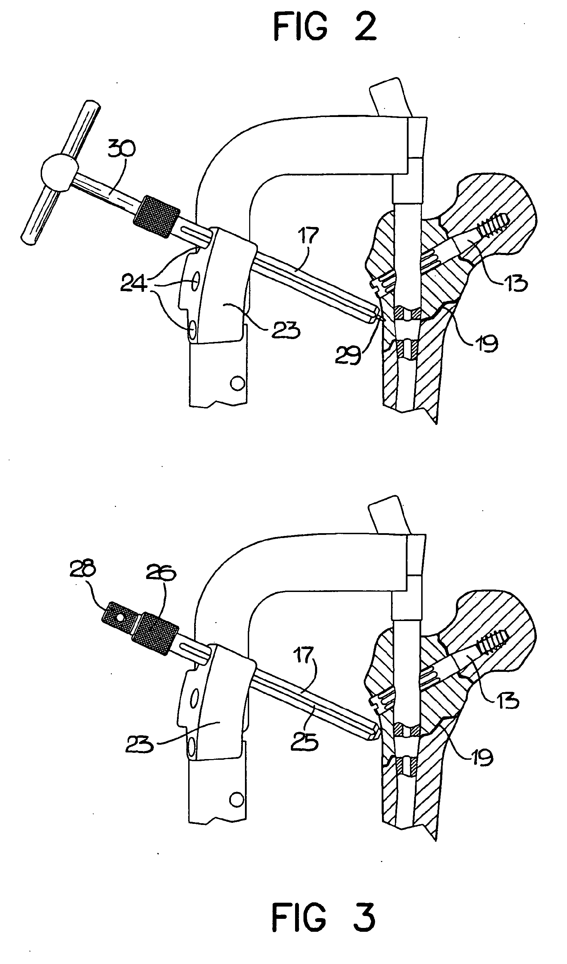

Figure 2 is a partially sectional detail front view of the device during the formation of a preliminary hole in the femur with a cortex penetration tool;
Figure 3 is a partially sectional detail front view of the same device during the insertion, in the sleeve, of a guiding cannula for the drilling tool;

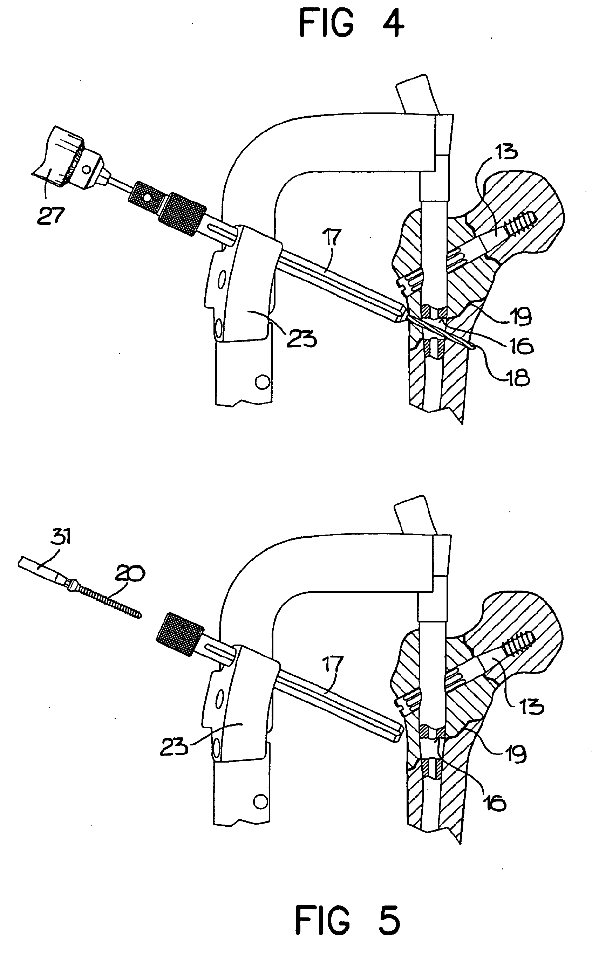

Figure 4 is a partially sectional detail front view of the device during the drilling of the femur through the fracture fissure and the intramedullary nail;
Figure 5 is a partially sectional detail front view of the same device during the insertion of the screw to be associated with the intramedullary nail;

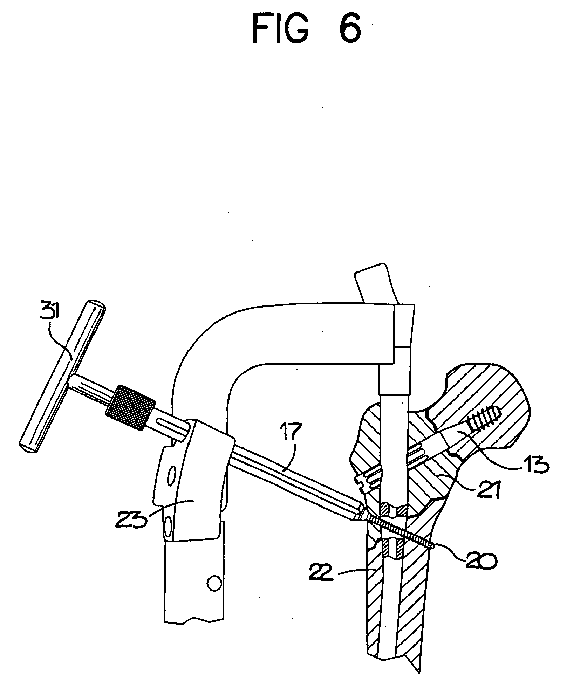

Figure 6 is a partially sectional detail front view of the device with the screw fully threaded into the femur through the slot of the intramedullary nail;
Figure 7 is a transverse sectional view, of the intramedullary nail, taken along the line VII-VII of Figure 1.

With reference to the figures, the reference numeral 1 generally designates a device for positioning a screw to be associated with an intramedullary nail inserted in the medullary canal of the femur according to the invention, particularly for treating fractures having a fissure located substantially in the subtrochanteric region.

The intramedullary nail 2, which can be any of the long or short type, has an upper end 3, which protrudes from the head 3a of the femur 4 and to which an arm 5 is connected; said arm has an L-shaped portion 6, which is arranged outside the limb of the patient and is extended by an end part that is substantially parallel to said nail 2.

The end part of the outer portion 6 of the arm 5 has, for example, means 7 for the insertion of distal screws for locking the intramedullary nail 2 at its lower end 8: such means 7 comprise, in a known manner, a plurality of parallel through holes 9 whose axes are substantially perpendicular to the axis of the nail 2, and a hollow stem 10, which can be inserted selectively in the holes 9 and in which at least one wire 11 for guiding a cannulated tool is inserted in order to produce one or more holes in the femur 4 for the engagement of the thread of the distal screws.

The intramedullary nail 2 has, substantially at its upper end 3, an oblique hole 12 to allow the passage of a head screw 13, which is implanted in a known manner along the protrusion 14 of the head 3a of the femur 4.

According to the invention, the positioning device comprises a supporting element 15, which is fixed at an adjustable level along the outer portion 6 of the arm 5, preferably so as to lie substantially at the same level as the head screw 13; moreover, a transverse slot 16 is provided at the upper end 3 of the intramedullary nail 2, below the oblique hole 12 for the head screw 13.

Furthermore, the longitudinal centerline plane of the slot 16 is inclined with respect to the axis of the head screw 13, which is inserted along the head 3a of the femur 4. The supporting element 15 is adapted to allow the selective orientation of a guiding sleeve 17 for a tool for perforating the femur 4, preferably a helical bit 18 of suitable diameter, in a number of directions that substantially converge toward the slot 16 formed in the nail 2; the slot 16, therefore, is adapted to allow the passage of the helical bit 18 in the optimum incident direction with respect to the fracture fissure 19, and then the passage of the screw 20 that provides the connection of the fragments 21, 22 formed by said fissure 19.

The supporting element 15 is constituted by a block 23, which is fixed along the outer portion 6 of the arm 5 and is affected by a plurality of through channels 24 having a circular cross-section for the insertion of the sleeve 17.

The respective axes of the through channels 24 are inclined along directions that substantially converge on the axis of the nail 2 at the centerline of the slot 16; for this purpose, the block 23 preferably has a convex curved shape, so that the axes of the channels 24 are co-planar and belong to the longitudinal centerline plane of the slot 16.

The sleeve 17, which has a slender tubular shape, has a longitudinal slot 25 for the engagement of locking means adapted to prevent the rotation of said sleeve 17 about its own axis; the sleeve 17 furthermore has, at the end where the helical bit 18 is inserted, a portion 26 having a larger diameter in order to stop and abut against the block 23.

The helical bit 18 of the femur 4 is locked and actuated by an adapted chuck 27 and is guided by a cylindrical cannula 28, which can be inserted in the sleeve 17.

Method of use of the device according to the invention is as follows: after determining the optimum direction along which the screw 20 is to be screwed in so as to connect the femur fragments 21 and 22 produced by a fissure 19 located in the subtrochanteric region, the sleeve 17 is inserted in the most appropriate through channel 24 among the channels formed in the block 23.

Then a preliminary hole 29 is formed in the femur 4, preferably by means of a suitable cortex penetration tool 30, through the sleeve 17, for subsequent drilling with the helical bit 18. After inserting the cannula 28 in the sleeve 17, the helical bit 18 is inserted in said cannula and perforates the femur 4 from side to side, passing through the slot 16 formed in the intramedullary nail 2; then the helical bit 18 is extracted and the screw 20 is screwed into the femur through the slot 16, for example manually by means of a screwdriver-like tool 31. The length of the screw 20 is determined with a suitable measurement device or, as an alternative, with a graduated visual scale on the helical bit 18.

The device allows to insert simply and easily a screw 20 through the femur 4 in order to reduce fractures having fissures 19 that are inclined at any angle with respect to the axis of the femur 2 and are located in awkward or scarcely accessible regions, such as for example the subtrochanteric region due to the possible presence of a head screw 13.

Moreover, the inclination of the longitudinal centerline plane of the slot 16 with respect to the plane of the head screw 13 avoids the risk of incurring in interference with said head screw during the insertion of the oblique screw 20 in the subtrochanteric region.

It has thus been found that the invention achieves the intended aim and objects.

The invention thus conceived is susceptible of numerous modifications and variations, all of which are within the scope of the appended claims.

All the details may further be replaced with other technically equivalent elements.

In practice, the materials used, as well as the shapes and dimensions, may be any according to requirements without thereby abandoning the protective scope of the appended claims.

The disclosures in Italian Patent Application No. BO20002A000200 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An intramedullary nail (2) with a bone fragment connection screw (20) and a femur head screw (13), in combination with a device (1) for positioning said bone fragment connection screw (20) connected with said intramedullary nail (2) for insertion in the medullary canal of the femur (4), said nail (2) having an upper end (3) for protruding from the head (3a) of the femur (4) and a lower distal end (8), said nail (2) having an oblique hole (12) provided at the upper end (3) thereof and said head screw (13) passing through said oblique hole (12), said device (1) comprising an arm (5) which is connected to the upper end (3) of the nail (2) and said arm (5) being provided with a portion for being arranged outside the femur (4) said device (1) further comprising a supporting element (15), which is fixed to said external portion (6) at an adjustable level and is adapted, due to a plurality of through channels (24), to allow the selective orientation of a guiding sleeve (17) for a tool (18) for drilling the femur (4) along multiple directions that converge toward said slot (16), which is formed in said nail (2) and is adapted to allow the passage of said tool (18) and said bone fragment connection screw (20) with the aim to easily reduce oblique fractures (19), **characterized in that** said device (1) further comprising means (7) at an external portion (6) of the arm (5) for inserting distal screws for locking the nail (2) at the lower distal end (8) thereof, said nail (2) further comprising a transverse slot (16) provided at said upper end (3) of said nail (2) below said oblique hole (12) for said head screw (13), said slot (16) having a longitudinal centerline plane which is inclined with respect to the axis of said head screw (13), and said bone fragment connection screw (20) passing through said slot (16).

2. The combination of claim 1, **characterized in that** said supporting element (15) is constituted by a block (23), which is fixed along said outer portion (6) of said arm (5) and is provided with a plurality of through channels (24) for the insertion of said sleeve (17).

3. The combination of claim 2, **characterized in that** said through channels (24) have respective axes that are inclined in directions that converge on the axis of said nail (2) at said slot (16).

4. The combination of claim 3, **characterized in that** said block (23) has a curved convex shape that is adapted to obtain the convergence of the axes of said through channels (24) in said slot (16).

5. The combination of one or more of the preceding claims, **characterized in that** said perforation tool is constituted by a helical bit (18), a guiding cannula for said bit (18), to be inserted in said sleeve (17), being provided.

## Patentansprüche

1. Ein Marknagel (2) mit einer Knochenfragmentverbindungsschraube (20) und einer Fumorkopfschraube (13), in Kombination mit einer Vorrichtung (1) zur Positionierung der mit dem Marknagel (2) verbundenen Knochenfragmentverbindungsschraube (20) zum Einführen in den medullären Kanal des Femur (4), wobei der Nagel (2) ein oberes Ende (3) zum Herausragen aus dem Kopf (3a) des Femur (4) und ein unteres distales Ende (8) aufweist, wobei der Nagel (2) eine schräge Bohrung (12) aufweist, die an dem oberen Ende (3) davon bereitgestellt ist, und wobei die Kopfschraube (13) durch die schräge Bohrung (12) führt, die Vorrichtung (1) einen Arm (5) umfassend, der mit dem oberen Ende (3) des Nagels (2) verbunden ist und wobei der Arm (5) mit einem Teil zur Anordnung außerhalb des Femur (4) bereitgestellt ist, die Vorrichtung (1) weiter ein Stützelement (15) umfassend, das an dem externen Teil (6) in einer justierbaren Höhe befestigt ist und angepasst ist, aufgrund der Mehrzahl von Durchgangskanälen (24), die selektive Orientierung einer Führungshülse (17) für ein Werkzeug (18) zum Bohren des Femurs (4) entlang mehrerer Richtungen zu ermöglichen, die in Richtung des Schlitzes (16) zusammenlaufen, der in dem Nagel (2) ausgebildet ist und angepasst ist, um das Durchführen des Werkzeugs (18) und der Knochenfragmentverbindungsschraube (20), mit dem Ziel Schrägfrakturen (19) bei weitem zu reduzieren, zu ermöglichen, **dadurch gekennzeichnet, dass** die Vorrichtung (1) weiter Mittel (7) an einem externen Teil (6) des Arms (5) zum Einsetzen von Distalschrauben für die Verriegelung des Nagels (2) an dem unteren distalen Ende (8) davon umfasst, der Nagel (2) weiter einen Querschlitz (16) umfassend, der an dem oberen Ende (3) des Nagels (2) unter der schrägen Bohrung (12) für die Kopfschraube (13) bereitgestellt ist, wobei der Schlitz (16) eine Längsachsenebene aufweist, die in Bezug auf die Achse der Kopfschraube (13) geneigt ist, und wobei die Knochenfragmentverbindungsschraube (20) durch den Schlitz (16) führt.

2. Die Kombination von Anspruch 1, **dadurch gekennzeichnet, dass** das Stützelement (15) von einem Block (23) gebildet ist, der entlang des äußeren Teils (6) des Arms (5) befestigt ist und mit einer Mehrzahl von Durchgangskanälen (24) für das Einführen der Hülse (17) bereitgestellt ist.

3. Die Kombination von Anspruch 2, **dadurch gekennzeichnet, dass** die Durchgangskanäle (24) jeweilige Achsen aufweisen, die in den Richtungen geneigt sind, die im Wesentlichen auf der Achse des Nagels an dem Schlitz (16) zusammenlaufen.

4. Die Kombination von Anspruch 3, **dadurch gekennzeichnet, dass** der Block (23) eine gebogene, konvexe Form aufweist, die angepasst ist, um die Konvergenz der Achsen der Durchgangskanäle (24) in dem Schlitz (16) zu erhalten.

5. Die Kombination von einem oder mehreren der vorhergehenden Ansprüche, **dadurch gekennzeichnet, dass** das Perforierungswerkzeug von einem schneckenförmigen Bit (18) gebildet ist, wobei ein Führungskanal für ein in die Hülse einsetzbares Bit (18) bereitgestellt ist.

## Revendications

1. Clou intramédullaire (2) avec une vis de raccordement de fragments osseux (20) et une vis de tête de fémur (13), en combinaison avec un dispositif (1) pour positionner ladite vis de raccordement de fragments osseux (20) raccordée audit clou intramédullaire (2) pour l'insertion dans le canal médullaire du fémur (4), ledit clou (2) ayant une extrémité supérieure (3) pour faire saillie à partir de la tête (3a) du fémur (4) et une extrémité distale inférieure (8), ledit clou (2) ayant un trou oblique (12) prévu au niveau de son extrémité supérieure (3) et ladite vis de tête (13) passant à travers ledit trou oblique (12), ledit dispositif (1) comprenant un bras (5) qui est raccordé à l'extrémité supérieure (3) du clou (2) et ledit bras (5) étant prévu avec une partie pour être agencé à l'extérieur du fémur (4), ledit dispositif (1) comprenant en outre un élément de support (15), qui est fixé sur ladite partie externe (6) à un niveau ajustable et est adapté, dû à une pluralité de canaux de passage (24), pour permettre l'orientation sélective d'un manchon de guidage (17) pour un outil (18) pour percer le fémur (4) le long de plusieurs directions qui convergent vers ladite fente (16), qui est formée dans ledit clou (2) et est adaptée pour permettre le passage dudit outil (18) et de ladite vis de raccordement de fragments osseux (20) avec le but de réduire facilement des fractures obliques (19), **caractérisé en ce que** ledit dispositif (1) comprend en outre des moyens (7) au niveau d'une partie externe (6) du bras (5) pour insérer des vis distales pour bloquer le clou (2) au niveau de son extrémité distale inférieure (8), ledit clou (2) comprenant en outre une fente transversale (16) prévue au niveau de ladite extrémité supérieure (3) dudit clou (2) au-dessous dudit trou oblique (12) pour ladite vis de tête (13), ladite fente (16) ayant un plan d'axe central longitudinal qui est incliné par rapport à l'axe de ladite vis de tête (13), et ladite vis de raccordement de fragments osseux (20) passant par ladite fente (16).

2. Combinaison selon la revendication 1, **caractérisée en ce que** ledit élément de support (15) est constitué par un bloc (23) qui est fixé le long de ladite partie externe (6) dudit bras (5) et est prévu avec une pluralité de canaux de passage (24) pour l'insertion dudit manchon (17).

3. Combinaison selon la revendication 2, **caractérisée en ce que** lesdits canaux de passage (24) ont des axes respectifs qui sont inclinés dans les directions qui convergent sur l'axe dudit clou (2) au niveau de ladite fente (16).

4. Combinaison selon la revendication 3, **caractérisée en ce que** ledit bloc (23) a une forme convexe incurvée qui est adaptée pour obtenir la convergence des axes desdits canaux de passage (24) dans ladite fente (16).

5. Combinaison selon une ou plusieurs des revendications précédentes, **caractérisée en ce que** ledit outil de perforation est constitué par un foret hélicoïdal (18), une canule de guidage pour ledit foret (18), destinée à être insérée dans ledit manchon (17), étant prévue.
